# EUROPEAN PATENT APPLICATION

(11) **EP 4 585 249 A1**
(43) Date of publication of application: **16.07.2025**
(21) Application number: 24151539.4
(22) Date of filing: 12.01.2024
(51) Int. Cl.: A61M 25/00, A61F 2/95

(54) **A CAPILLARY-FORCE BASED APPROACH TO REDUCING THE NEED FOR FLUSH SUPPORT ON LUMENS IN CATHETER SYSTEMS**

(71) Applicant: BIOTRONIK SE & Co. KG, 12359 Berlin (DE)
(72) Inventor: Taff, Brian M., Portland, 97217 (US)
(74) Representative: Biotronik Corporate Services SE

(57) **Abstract**

The present application relates to a catheter, which may comprise an inner shaft and an outer shaft such that a lumen is defined therebetween, wherein the lumen comprises a distal opening and a proximal opening, wherein the proximal opening is configured such that a gas in the lumen can leave the outer shaft via the proximal opening and wherein the proximal opening is configured to be impermeable for a liquid.

## Description

The present application relates to a catheter which may reduce the need for flush support on lumens in catheter systems.

Catheters are known as standard tools for determining and treating coronary diseases. Catheters typically comprise extended tubular-like systems which may be inserted into a blood vessel of a patient and may be pushed to a certain region in the coronary system of the patient such that desired diagnostics or treatments can be performed.

The demands on a catheter may be versatile such that a variety of different catheters have been developed to meet the respective (medical) requirements. Catheters may relate to balloon catheters, e.g., to widen an at least local stenosis, or may be used for implantations of a medical device, e.g., a stent, an intracardiac pacemaker or an implantable pressure sensor, etc. Especially, if a catheter is used to transport a medical device to a certain location in the blood vessel or in the heart of a patient, the catheter may be provided with an inner lumen in which the medical device to be delivered may be stored. In some cases, a catheter may not be limited to a single lumen but may also be provided with multiple lumina.

In particular, if catheters are provided with at least a distal opening, e.g., if the catheter may be used for an implantation of a medical device, it may be required to rinse the catheter. Said rinsing may be required to remove air before introducing a catheter to the circulatory system of the patient and/or to enable the removal of stagnant, retained blood volumes that may risk the shedding of thrombi within the circulatory system of the patient.

Said rinsing may be performed by providing the catheter with a dedicated flushing port by means of which a rinsing of the respective lumen may be facilitated. If a multiple lumen catheter is used, each lumen may be flushed with a dedicated and separate flush port. However, the use of a dedicated flush port for each in-catheter lumen may introduce mechanical encumbrances that directly compete with efforts to optimize user comfort when operating such systems as the catheters' handles tend to be bulkier and their control placement and accessible ranges tend to be constrained/impacted.

Catheter manufacturers have approached efforts to reduce the number of flush ports needed in their designs using a variety of approaches. However, such implementations oftentimes require a potential handle of the catheter to become rather bulky and their control placement and accessible ranges tend to be constrained/impacted.

The dominant challenge associated with connecting multiple in-catheter lumina by means of a single flush port stems from the resultant resistively-balanced network of flows. Within such a framework, ensuring ample or sufficient fluid flushing flow within a specific lumen becomes highly dependent upon the ability to stably maintain the entire balanced network of connections. Such balancing can be impacted by restrictions that may emerge in the connections between individual lumens as a result of product use. In other words, if a blood clot clogs a fluidic pass-through connection between two lumens, it can completely halt fluid ingress into the non-primary lumen and alter the balance of intended fluid flows in the remaining portions of the flushed network. In a sense, such designs are viable as long as they are not clogged, but the point of catheter lumen flushing is often to remove clogs. As a result, such designs are critically susceptible to fouling with use wherein their operation is sufficient at the start of a patient procedure but degrades over the course of the procedure. Further, designs that employ stylets to "shift gears" (e.g., known from US 10,405,907) within the catheter's internal fluid network and direct fluid flush flow in deliberate fashion demand that the user operate a separate stylet and that the catheter design be orchestrated around such design elements often limiting their use in specific applications and rendering them rather difficult to use.

In view of the aforementioned drawbacks associated with the flushing of catheter systems, there is therefore a need at least partially overcome these known drawbacks by improving catheters such that an enhanced, easier to operate or more reliably rinsing may be facilitated.

This need may at least in part be met by the aspects described herein.

A first aspect relates to a catheter which may comprise an inner shaft and an outer shaft such that a lumen is defined therebetween. The lumen may comprise a distal opening and a proximal opening and the proximal opening may be configured such that a gas in the lumen can leave the outer shaft via the proximal opening. Moreover, the proximal opening may be configured to be impermeable for a liquid.

Such a catheter design allows a flushing of at least a portion of the lumen defined between the inner shaft and the outer shaft while maintaining a rather simple and easy to handle catheter design as no undue operating overhead is required. In particular, no additional shafts (and general any external support accessories such as, e.g., syringes, (dangling attached) tubes, etc.) may be required to perform a rinsing task. In particular, gas may easily be removed from the lumen via the proximal opening. No stylets or other accessory components are required to set the catheter to a desired rinsing state without gas inside the lumen for example. This may in particular contribute to decreased cognitive overhead to be supplied by the medical staff, which may operate the catheter, and may focus on the medical intervention itself without having to be overly focused on a complicated catheter handling (such as it may arise from, e.g., the stylets). Since the catheter according to the first aspect of the invention provides a simplified catheter design, less components need to be assembled which may lead to the advantageous effect of a cost-reduction in the manufacturing process of such a catheter. A further advantageous effect can be seen in a decrease in volume of the catheter to support its core functionality.

A catheter, in the context of the present application, may relate to any tubular structure which may be inserted into the vascular/circulatory system (e.g., a blood vessel or the heart) of a patient. In a preferred implementation, such a catheter may be provided with a certain flexibility (e.g., bendability, ductility, stretchability, etc.) such that the catheter may be pushed, preferably from outside of the patient's body, into a certain region/location in the vascular system of the patient. Moreover, these properties of the catheter may also allow that the catheter may be retractable from the respective location in the body of the patient.

A distal side may be understood as relatively close to one end of the catheter (as seen with respect to a longitudinal (length) extension of the catheter) which may be farthest away from an operator of the catheter (e.g., a physician), e.g. a tip portion of the catheter which may be closest to the center of the body of the patient (e.g., the end of the catheter which may be inserted into the body of the patient first). Contrary to the distal side, a proximal side may be understood as being relatively close to the operator of the catheter (e.g., outside of the body of the patient or at least close to the surface of the body of the patient). The proximal side of the catheter may be located opposite to the distal side of the catheter (with respect to a longitudinal extension (i.e., a length direction) of the catheter).

The inner shaft may be arranged in the outer shaft in a concentrical configuration, i.e., the inner shaft may be arranged symmetrically within the outer shaft such that the distance, measured along a radial direction, from any point on an outer side of the inner shaft to a point on an inner side of the outer shaft may be equal. Alternatively, it may also be possible that the inner shaft is located closer to one side of the outer shaft. In other words, the inner shaft may be located closer to a right side of the outer shaft as compared to a respective opposing left side of the outer shaft (with respect to a cross sectional view, transverse to a longitudinal extension of the catheter).

The distal opening may be configured such that a fluid can enter the lumen via the distal opening.

The opening may be understood as an opening which may always be set to an open state (similar to, e.g., one end of a tube) or which may be opened or closed (e.g., by means of a sliding mechanism which may be operable by an operator of the catheter and which may at least partially be arranged in a tip portion of the catheter on the outer shaft) according to the current needs.

Even though different cross sections may be possible (e.g., a rectangular cross section), the opening may preferably be provided with a circular and/or ring-shaped cross section (as seen from a transverse, relative to the longitudinal extension of the catheter, imagined cut through the catheter).

Particularly, the dimensions of the distal opening is defined by an inner diameter of the outer shaft, particularly by an inner diameter of a distal part of the shaft. Particularly, the distal part of the outer shaft may exhibit a larger inner and/or outer diameter than a proximal part of the outer shaft, for example in case of when the distal part of the outer is configured to receive or sheath an implantable medical device, e.g., an intracardiac pacemaker or an implantable pressure sensor.

A fluid may be understood as a gas (e.g., air, oxygen, etc.) or as a liquid (e.g., water, any kind of solutions which may be beneficial for a catheter-based intervention, etc.). In some scenarios the fluid may also be blood which may enter the distal opening of the lumen as a result of the catheter being inserted into a blood vessel of the patient.

By allowing fluid ingress through the distal opening of the catheter, the lumen may be filled with the fluid. As a result, any potential air residing in the lumen, may be superseded in a direction which is free of the fluid, e.g., in a direction of the proximal opening. Therefore, the risk that air may escape into the blood vessel of the patient, which may cause harmful effects therein (e.g., a coagulation), may be prevented.

Moreover, a distance between the inner shaft and the outer shaft may be sufficiently small such that a liquid at the distal opening may be pulled into the lumen in a proximal direction.

It may be possible that the liquid is pulled into the lumen by a surface tension of the liquid (e.g., caused by cohesion within the liquid) and/or adhesive forces between the liquid and the outer side of the inner shaft and the inner side of the outer shaft. As a result, the liquid may form a meniscus in between the outer wall of the inner shaft and the inner wall of the outer shaft. In some cases, the pulling of the liquid into the proximal direction may be based at least in part on capillary forces.

The required (radial) separation between the inner shaft and the outer shaft to allow for a pulling of the liquid may depend on the respective liquid and may be adapted such that only a certain liquid out of a variety of liquids which may reside in a distal volume of the lumen may be pulled into the lumen in a proximal direction. In other words, the separation may be adapted such that only blood may be pulled in the proximal direction whereas, e.g., water may not be pulled in the proximal direction.

By providing the inner shaft in the outer shaft in such a manner that the separation between the two shafts it is sufficiently small to allow a liquid to be pulled into the lumen in a proximal direction, it may be facilitated that even a proximal portion of the catheter, and more specifically the lumen defined between the inner shaft and the outer shaft, may be filled with a desired liquid. The filling may in particular be facilitated without the requirement for any further external input by, e.g., the operator of the catheter or any additional hardware requirements (such as, e.g., a vacuum unit). Therefore, a simplified handling of the catheter and a respective cost-efficient production (as, e.g., no valve systems are required to control the flow if the liquid) of the catheter may be provided.

In some cases, the liquid may be a rinsing liquid.

The rinsing liquid may be adapted such that it may disinfect (e.g., if the liquid is a disinfectant) the walls surrounding the lumen in which it resides and/or to supersede any blood residing in the lumen (e.g., if the liquid is water (and/or a saline solution) and/or a heparin liquid (e.g., a heparinized saline)) and/or to supersede any air residing in the lumen (e.g., if the liquid is any or a combination of the aforementioned liquids).

The rinsing liquid may additionally or alternatively be adapted such that it may mitigate a blood coagulation potential (e.g., if the liquid at least contains a heparin liquid).

By using a rinsing liquid, a flushing of the lumen may be facilitated and undesired remnants (e.g., blood coagulations, air) residing in the lumen (e.g., in a proximal portion of the lumen) may be flushed and thus removed. The flushing may be performed prior to an insertion of the catheter into the body of the patient and/or once when the catheter has been inserted into the body of the patient and/or the catheter may be flushed repeatedly (e.g., every 5 min, every 10 min, etc. and/or after every treatment step, etc.) when inserted into the body of the patient (e.g., to avoid a blood coagulation during the treatment).

The catheter may further be adapted such that the rinsing liquid can be delivered into the lumen by insertion into an inner lumen of the inner shaft.

In some cases, the inner shaft of the catheter may be hollow such that the inner shaft may be provided with an inner lumen. The inner lumen of the inner shaft may be used to insert the rinsing liquid from a proximal direction of the catheter (e.g., the rinsing liquid may be inserted into the inner lumen of the inner shaft by an operator of the catheter).

Therefore, a simplified design for a flushable catheter may be provided which does not require any additional tubes and/or shafts for providing a flushing liquid to a distal region of the catheter. This may increase the ease of use and may also decrease the manufacturing costs of the catheter (e.g., as less parts and less (complicate) manufacturing steps are required).

The catheter may be adapted such that the rinsing liquid at least partially reduces the inflow of blood into the lumen. For example, if the rinsing liquid at least partially accumulates or flows into certain regions of the lumen and resides therein, blood may not reside in said regions. Therefore, an undesired inflow of blood into certain regions of the lumen may be avoided. In some cases, it may also be possible, to entirely fill the lumen with the rinsing liquid such that no inflow of blood is allowed.

Therefore, a controlled inflow of blood into the lumen may also be facilitated at least by providing a respective volume of the rinsing liquid in the distal portion of the catheter which will then reside in certain regions of the lumen (e.g., in a distal-most part of the catheter and/or partially along a proximal direction of the lumen). Such an implementation allows variable amounts of blood entering the catheter without the requirement for additional control overhead (e.g., such as valves, etc.).

An outer side of the inner shaft and/or an inner side of the outer shaft may be hydrophilic. In some cases, the material from which at least the outer side of the inner shaft and/or the inner side of the outer shaft is made may be hydrophilic by itself. In some cases, it may also be possible that any material, from which at least the outer side of the inner shaft and/or the inner side of the outer shaft is made, may not intrinsically be hydrophilic but may be provided with hydrophilic properties by coating the respective surfaces of the material with a hydrophilic coating accordingly to establish hydrophilic properties.

Providing at least the outer side of the inner shaft and/or the inner side of the outer shaft in a hydrophilic manner, the tendency of a fluid to flow and/or climb along a surface of the shafts (which may be hydrophilic) may be increased (even against gravity). Therefore, an improved pulling of a liquid, e.g., a rinsing liquid into certain regions of the lumen may be facilitated (e.g., in proximal direction of the lumen) and additionally be promoted (e.g. in addition to capillary forces). Therefore, also regions which may be rather difficult to be accessed by the rinsing fluid may thus be accessible and/or be flushable by, e.g., the rinsing fluid. Moreover, the hydrophilic properties may also allow a superseding of air remnants which may reside on the hydrophilic surfaces as the hydrophilic properties may lead to an increased preference for liquids to undergo addition to a hydrophilic region as compared to air. Therefore, an increased tendency for a replacement of air by a liquid may additionally be promoted.

The inner shaft and the outer shaft may be movable relative to each other at least along a longitudinal extension of the inner shaft and the outer shaft. Additionally or alternatively, the inner shaft may also be movable transversely to a longitudinal extension of the inner shaft and the outer shaft. In some cases, the inner shaft may also be movable in a radial direction such that the distance from the inner shaft to the outer shaft may be shorter in one direction as compared to a radial opposing direction.

By providing the inner shaft movable relative to the outer shaft (and vice versa), it may be facilitated that the inner shaft may, e.g., be moved back and forth (with respect to a length direction of the catheter) within the outer shaft. Therefore, a distal tip of the inner shaft may be placed at different locations within the outer shaft and the location at which a potential rinsing fluid may exit the inner lumen of the inner shaft may be adjustable. This may beneficially allow a fine tuning of the regions of the lumen to be rinsed.

The catheter may be configured such that a proximal flow of liquid within the lumen may cause gas in the lumen to be pushed out of the lumen via the proximal opening. In some cases, the proximal opening may be located outside of the body of the patient.

The proximal flowing liquid may for example be blood (e.g., inflowing from the distal opening) or a rinsing fluid, e.g., provided by means of the inner lumen of the inner shaft and/or the distal opening.

As a result of the proximal flow of liquid, the liquid may supersede gas (e.g., air), which may reside in the lumen, along the proximal direction and may thus push the gas even further in a proximal direction to the proximal opening at which the gas may (be forced to) leave the catheter.

The proximal opening may comprise a venting structure, preferably a one-way venting structure.

In a preferred case, the venting structure may relate to any structure (e.g., implemented as a dedicated membrane) which may allow gas to exit the proximal end of the catheter, but which prohibits any air from entering the proximal end of the catheter and thus from further propagating towards a distal end of the catheter. In other words, a gas flow may only be permitted in one direction.

In a preferred case, such a venting structure may, e.g., be a one-way check valve and/or any semi-permeable membrane.

A venting structure may at least contribute to the avoidance of any harmful effects occurring to the health state of the patient which may arise from a gas (e.g., air) resident in the lumen of the catheter propagating into a blood vessel of the patient in which it may cause coagulation and/or a thrombosis.

A distance between the inner shaft and the outer shaft may be adapted such that a blocking of the lumen by stagnant blood may be avoided. In a preferred case, the (radial) distance between the outer side of the inner shaft and the inner side of the outer shaft may be adapted such that a coagulation of the blood may be avoided when entering the respective lumen between the inner shaft and the outer shaft. Such a coagulation may, e.g., arise from slowing down inflowing blood and a resulting increase in viscosity.

Therefore, a contribution to the avoidance of a blocking of the lumen may be provided, by at least partially ensuring that a gas may exit the lumen in a proximal direction prior to a catheter-based treatment and/or during a catheter-based treatment. Moreover, it may further be ensured that the inner shaft remains movable relative to the outer shaft as an adhering of the outer shaft relative to the inner shaft, which may arise from a blood coagulation (e.g., stagnant and/or retained blood), may be avoided.

The proximal opening may reside in a handle of the catheter. In some cases, the proximal opening may be located in the handle of the catheter, wherein the handle may provide means for securely being holdable (e.g., a grooved surface for improved ergonomic handling) by the operator of the catheter. The handle of the catheter may thus preferably be located outside of the body of the patient.

By providing the proximal opening in the handle of the catheter, it may be ensured that that the proximal opening may always be located outside of the body of the patient. Moreover, it may be ensured the proximal opening is covered by the handle housing such that the proximal opening may not accidentally be blocked, e.g., by dust. Moreover, by arranging the proximal opening in the handle, operator access may be denied (which may block the proximal opening) and the proximal opening may be arranged such that it may be invisible for the operator thus further contributing to the aforementioned beneficial advantage.

The proximal opening may comprise a polymer. The polymer may comprise polyurethan (PU) and/or polytetrafluorethylene (e.g., Teflon). The proximal opening may comprise a (woven) fabric and/or a membrane, e.g., comprising the polymer. In a preferred implementation, the fabric and/or the membrane may be made from a material which is known under the brand name Gore-Tex.

In particular, by manufacturing the polymer at least in part from Gore-Tex, it may be facilitated that gas residing in the lumen may exit the lumen whereas the Gore-Tex material may be impermeable (e.g., for blood and/or saline liquids) for any liquids in the lumen. Therefore, an advantageous separation of liquid and gas may be provided by keeping the design of the catheter simple and cost-efficient.

The outer shaft may be configured as an outer surface of the catheter and/or the lumen may be adapted to be directly adjacent to an inner lumen of the inner shaft.

If the outer shaft is configured as an outer surface of the catheter, the outer surface of the outer shaft is essentially perceivable as the outer surface of catheter itself. In this case, no additional shaft/tube or coating may be present covering the outer shaft. Hence, manufacturing time and costs as well as the weight of the catheter may be reduced.

In some cases, the lumen may directly be adjacent (i.e., without any further components in between) to an inner lumen of the inner shaft such that, e.g., the inner lumen of the inner shaft may directly end in the lumen.

The catheter may further be adapted to accommodate a medical device (e.g., an active medical device) distally to the lumen.

In some cases, at least the inner diameter of at least the outer shaft may be adapted such that it may circumscribe a medical device. A medical device may, e.g., be any kind of medical apparatus which may allow for a medical treatment and/or a medical diagnostic and which may need to be delivered to a certain location in the vascular system of the patient.

If the catheter is adapted to accommodate a medical device, the catheter may be used as a delivery system for, e.g., implants which may thus increase the spectrum of potential applications of the catheter.

A second aspect of the application relates to a catheter which may comprise one or more of an inner shaft, an outer shaft, and a sealing element. The catheter may be adapted to comprise a first predetermined configuration in which the sealing element may be arranged to seal a distal end of the inner shaft and wherein the catheter may be adapted to comprise a second predetermined configuration in which the sealing element may not be arranged to seal the distal end of the inner shaft. The inner shaft may comprise a pressure sensitive valve.

It is noted that features of the second aspect as described herein may also be combinable with features of other aspects of this application (e.g., with features of the first aspect and/or with features of a third aspect described further below and/or with features of a fourth aspect as will further be explained below).

The sealing element may be understood as any suitable element which may (reversible) seal the inner shaft.

By adapting the catheter such that it may comprise the two predetermined configurations as mentioned above, a selective opening of a distal portion of the catheter may be facilitated. Notably, in the first predetermined configuration, a selective setting of the inner shaft to an open and/or closed state may be facilitated according to the current needs of a catheter-based treatment.

An inner lumen of the inner shaft may be in fluid communication with a distal portion of the inner lumen of the outer shaft, when the catheter may be in the second predetermined configuration, such that the distal portion can be rinsed via the inner lumen of the inner shaft.

The fluid communication may be arranged such that a fluid which may be inserted into the inner lumen of the inner shaft from a proximal end of the catheter may be delivered to a distal end of the inner lumen of the inner shaft and delivered to the distal portion. Therefore, a selectable rinsing of a distal portion of the catheter may be facilitated when needed.

The catheter may comprise means for setting the first predetermined configuration and/or the second predetermined configuration from a proximal end of the catheter.

The means for setting may relate to a mechanical system which may be initiated to set the catheter into the first predetermined configuration or the second predetermined configuration, e.g., by means of actuating the sealing element such that it may be set into a state which may be associated with the first predetermined configuration or the second predetermined configuration of the catheter. The mechanical system may receive a respective command for setting the catheter into the first predetermined configuration or the second predetermined configuration. For example, a respective wire-like element may be arranged in the inner lumen of the inner shaft.

Additionally or alternatively, the means for setting may relate to an attachment means (e.g., a hook-like structure) by means of which the catheter may be set into the first predetermined configuration or the second predetermined configuration.

By allowing that the catheter may be set into the first predetermined configuration and/or the second predetermined configuration from a proximal end of the catheter, a "remote operability" of the distal portion of the catheter may be facilitated. Therefore, the distal portion of the catheter may be adapted to the current needs of the catheter-based treatment and do not have to be prepared prior to the treatment.

The sealing element may comprise a manipulator arranged in an inner lumen of the inner shaft and may extend along a proximal direction in the inner lumen of the inner shaft.

The manipulator may relate to any means which may be adapted to transfer an operation from a proximal portion of the catheter to the sealing element, e.g., initiated/executed by an operator of the catheter, to the sealing element. Said operation may be a force transfer (e.g., if the manipulator is implemented as a wire-like element which may provide sufficient bending resistance such that a force may be transferred from a proximal portion of the catheter, along the wire and to the sealing element). If the manipular is implemented as a wire-like element, the sealing element may, e.g., be pushed in a distal direction (thus setting the catheter in the second predetermined configuration) or may be retracted in a proximal direction to seal the inner shaft (thus setting the catheter in the first predetermined configuration).

The manipular may allow a transfer of a command to be delivered to the sealing element from a proximal end of the catheter and may therefore contribute to the "remote operability" of the rinsing of a distal end of the catheter. By arranging the manipular in the inner lumen of the inner shaft, the manipulator may be protected by the inner shaft such that the manipulator may not be damaged during a potential pushing and/or stirring of the catheter to a desired location in the body of the patient.

The catheter may be adapted such that the inner shaft and the sealing element can be moved relative to each other by applying a force via the manipulator.

Since the manipular may be attached to the sealing element, it may be possible to hold the sealing element spatially fixed at a certain location (e.g., by applying a pulling force onto the manipulator oriented along a proximal direction). By pushing the inner shaft (e.g., from a proximal direction of the catheter to a distal direction of the catheter) the inner shaft may be moved relative to the sealing element.

By adapting the sealing element and the inner shaft such that they are movable relative to each other, a simplified and retractable (tether-based) sealing mechanism may be facilitated which is only based on a minimized number of components. Therefore, the handling of the catheter may be simplified.

The sealing element may further comprise a funnel-like element in fluid communication with the inner lumen of the inner shaft by means of which the catheter may be set into the first predetermined configuration or the second predetermined configuration.

A funnel-like element may be understood as a sealing component which is provided with a radius which extends (e.g., linearly) along the distal direction (i.e., the funnel may be provided with a minimum radius in a portion which is closest to the inner shaft and may extend in radius towards the distal portion of the catheter).

The funnel-like element may be provided with an opening on a distal side of the funnel-like element and on a proximal side of the funnel-like element such that the funnel-like element may at least be in fluid communication with the inner lumen of the inner shaft.

By providing the sealing element with a funnel-like element, an at least liquid tight sealing of the inner shaft may be facilitated. The sealing may, e.g., be achieved by contacting a distal portion of the funnel-like element to a sealing partner which is not permeable to a liquid (e.g., any kind of plugs, a medical device, etc.), whereas the proximal portion of the funnel-like element may be attached to a distal end of the inner shaft. Therefore, a simplified and easy to handle sealing mechanism may be facilitated.

Another, third aspect of the present application relates to a catheter which may comprise an inner shaft and an outer shaft and may comprise a medical device arranged within the outer shaft. The catheter may be adapted to comprise a first predetermined configuration in which the medical device may be arranged to seal a distal end of the inner shaft.

It is noted that the third aspect of the application described herein may also be combined with features of other aspects of this application (e.g., with features of the first aspect of the application and/or with features of the second aspect of the application and/or with features of the fourth aspect of the application as will further be explained below).

The medical device may be a stent (e.g., non-expanding or a self-expanding stent, etc.) or an active medical device (e.g., a (leadless) pacemaker), etc. In some cases, the medical device may only temporarily (e.g., for a certain investigation and/or check-up and/or a short-term treatment) be inserted into the body of the patient. In some cases, the medical device may permanently be inserted into the body of the patient.

The medical device may be arranged to seal the distal end of the inner shaft by liquid-tightly and/or gas-tightly joining the medical device and the distal end of the inner shaft without any further sealing means (such as, e.g., rubber elements which may be placed in between the medical device (e.g., at a proximal side of the medical device) and the distal end of the inner shaft). Alternatively, an additional sealing means may be placed in between the medical device and the distal end of the inner shaft.

The third aspect of the application may thus allow a simplified sealing mechanism for the inner shaft. Since the medical device in the outer lumen may be adapted to seal the inner shaft, no additional sealing components (and in particular no mechanical sealing components such as, e.g., valves) are required. This advantageously contributes to a simplified catheter design and a simplified operability of the catheter.

The catheter may further be adapted to comprise a second predetermined configuration, in which the medical device may not arranged to seal the distal end of the inner shaft.

In the second predetermined configuration, the medical device may be moved to a more distal position in the outer shaft as compared to the location of the medical device in the first predetermined configuration such that a gap between the distal end of the inner shaft and the proximal end of the medical device may be generated. Therefore, in the second predetermined configuration, the inner shaft may not be sealed.

By providing the catheter with a second predetermined configuration into which the catheter may be set, a selective and simplified sealing mechanism for the inner shaft may be provided. Therefore, an easy-to-handle sealing mechanism, which may be operated during a catheter-based intervention, may be facilitated.

The inner shaft may be provided with a pressure sensitive valve.

The pressure sensitive valve may preferably be located on a more proximal portion of the inner shaft (e.g., compared to the sealing element and/or the medical device) and may, e.g., be implemented as a one-way check valve.

In some cases, the inner shaft may be provided with a single pressure sensitive valve. Alternatively, the inner shaft may also be provided with more than one pressure sensitive valve. If the inner shaft is provided with more than one pressure sensitive valve, the pressure sensitive valves may be located at a same longitudinal position on the inner shaft. Additionally or alternatively, the more than one pressure sensitive valve may also be located along a longitudinal direction of the inner shaft and (equally) spaced apart from each other.

Providing the inner shaft with at least one pressure sensitive valve, a situational opening of the valve, located on the inner shaft, may be facilitated.

The pressure sensitive valve may be configured to allow fluid communication between an inner lumen of the inner shaft and an inner lumen of the outer shaft, if the pressure in the inner shaft exceeds a predefined value.

The pressure at which the at least one pressure sensitive valve may allow a fluid communication may be preset prior to the catheter-based treatment. However, in some cases, the respective pressure may also be settable from outside the patient's body during a catheter-based treatment.

If more than one pressure sensitive valve is provided, it may be possible that all pressure sensitive valves allow a fluid communication if the same pressure is exceeded. Alternatively, it may be possible that the pressure sensitive valves are set to different pressure values at which they may allow a fluid communication.

The increase of pressure in the inner shaft may be promoted by setting the catheter into the first predetermined configuration and by additionally filling the inner lumen of the inner shaft with a fluid (e.g., a rinsing liquid).

By providing the inner shaft with at least one pressure sensitive valve, an automatic opening of the at least one pressure sensitive valve may be facilitated without any further input from an operator of the catheter. Therefore, the handling of the catheter may be simplified.

As a result of the fluid communication, a lumen may be defined in between the inner shaft and the outer shaft which can be rinsed from a proximal direction of the catheter towards a distal direction of the catheter.

The at least one pressure sensitive valve may preferably be located at a more proximal portion of the inner shaft as compared to a distal tip of the inner shaft. The at least one pressure sensitive valve may further be adapted such that it preferably allows a fluid communication in a distal direction of the catheter, i.e., if the inner lumen of the inner shaft is provided with a rinsing liquid, the rinsing liquid in the inner lumen of the inner shaft may preferably exit the inner shaft such that the rinsing fluid flows in the lumen in a distal direction.

Since the at least one pressure sensitive valve may preferably be located on a more proximal portion of the inner shaft (as compared to a distal tip of the inner shaft), a rinsing of the lumen defined in between the inner shaft and the outer shaft may be facilitated from a more proximal direction (which may not be rinsed if the catheter is set to the second predetermined configuration). Therefore, a longer proximal portion of the catheter may be rinsed.

The medical device may comprise an attachment means.

The attachment means may relate to a hook-like structure, a glued connection, a hook and loop fastener, etc. such that the medical device may be engageable with a contact partner.

By providing the medical device with an attachment means, an interaction of the medical device with a contact partner may be facilitated.

The catheter may further comprise a manipulator, wherein the manipulator is attached to the attachment means and extends along a proximal direction in the inner lumen of the inner shaft.

The manipular (acting as a contact partner for the medical device) may be engageable with the attachment means of the medical device. The manipular may preferably be a wire-like structure to allow a manipulation of the medical device from a proximal direction of the catheter (e.g., from outside the patient's body).

By providing the catheter with a manipulator, a simplified manipulation of the medical device may be facilitated from a proximal direction of the catheter ("remote operability") and wherein the manipulator may further be protected by the inner shaft from damages as a potential result from operating the manipulator from the proximal direction of the catheter.

The medical device may be movable relative to the inner shaft as a result of applying a force, along a longitudinal axis of the catheter, onto the manipulator thereby allowing setting the catheter into the first predetermined configuration and/or the second predetermined configuration.

Therefore, a simplified and easy to use manipulation of the position of the medical device, relative to the inner shaft, may be allowed. Moreover, also a setting of the catheter into the first predetermined configuration or the second predetermined configuration, may be simplified at least for the aspect that no additional components are required since the medical device may reside in a distal portion of the catheter regardless from other components of the catheter.

The catheter may be a delivery system.

In some cases, the medical device may be delivered (e.g., for residing permanently in the vascular system of the patient), by the catheter, to a certain location in the vascular system of the patient.

By adapting the catheter as a delivery system, it may be facilitated that the overall simplified and easy to handle catheter (as described herein) may be used for the delivery of medical devices. Therefore, also the delivery of medical devices may be simplified and improved as compared to delivery systems known from the art.

A fourth aspect of the application relates to a catheter which may comprise an inner shaft and an outer shaft arranged such that a lumen may be defined therebetween. The catheter may further comprise at least one blocking element which may be arranged on an inner side of the outer shaft and/or on an outer side of the inner shaft and wherein the at least one blocking element may be adapted such as to stop blood from flowing past the at least one blocking element in a proximal direction, but to allow gas to flow past the at least one blocking element in the proximal direction.

It is noted that features of the fourth aspect as described herein may also be combinable with features of other aspects of this application (e.g., with features of the first aspect of the application and/or with features of the second aspect of the application and/or with features of the third aspect).

By providing the catheter with a blocking element as described beforehand, a separation between a liquid and gas residing in the lumen may be facilitated. Therefore, gas residing in the lumen may be removable or may be maintained in a notionally entrapped condition.

The at least one blocking element may be adapted to stop a blood gas meniscus moving in the proximal direction or in the distal direction.

A blood gas meniscus may be understood as a mixture of blood and gas (preferably air which may have been resident in the lumen), such that a blood gas meniscus has been formed. The gas may have at least partially contributed to a coagulation of the blood.

The blood gas meniscus may possess the potential to block certain portions, in particular those portions of the catheter which may have a narrow (radial) width. In particular, the blood gas meniscus may tend to at least partially block the lumen in the proximal portion of the catheter such that in some cases a sufficient passing of gas towards a further proximal direction may not be guaranteed any more.

The at least one blocking element may be textured.

At least the surface of the at least one blocking element may further be adapted with a texture and/or molded. The texture may preferably be a roughening of the surface, e.g., by providing the at least one blocking element with one or more recesses and/or one or more protrusions.

By providing the at least one blocking element with a texture, the at least one blocking element may thus be provided with an increased adhesion to decrease the risk that a blood gas meniscus may accidentally disengage from the at least one blocking element.

Advantageously, the texture may be adapted to prevent the blood gas meniscus from moving in the proximal direction, and/or in the distal direction.

The texture may be adapted such that it provides increasing adhesion properties for the blood air meniscus along a proximal longitudinal direction of the catheter (e.g., by increasing the roughness of the surface of the at least one blocking element). In such a case the tendency of the blood gas meniscus for being trapped by the at least one blocking element increases the further the blood gas meniscus may move in a proximal direction.

By adapting the texture such that it may prevent the blood gas meniscus from moving in the proximal direction, the risk of a blocking of the lumen in a proximal portion of the catheter may further be reduced (e.g., in non-flushable regions of the catheter).

The blood air meniscus may be nominally compressed as a result of the blood gas meniscus being pushed in the proximal direction by blood entering the catheter through a distal opening.

Since the blood air meniscus may preferably be trapped by the at least one blocking element, a further movement of the blood air meniscus in the proximal direction and/or in the distal direction may be prevented. However, since blood may enter the catheter from a distal opening of the catheter, a pressure in the distal portion of the catheter may increase and may evolve a pressure-dependent pushing force in the proximal direction of the catheter (as the proximal portion of the catheter may have a lower pressure pressure). Since the blood gas meniscus may be trapped by the at least one blocking element (and can thus not be pushed in the proximal direction or in the distal direction), the blood gas meniscus may undergo a nominal compression which may decrease the size of the trapped blood gas meniscus.

By nominally compressing the blood gas meniscus, the size of the blood gas meniscus may be decreased. Therefore, the spatial requirements (e.g., a size) of the blood gas meniscus may also be decreased. This may in particular allow that multiple, separate blood gas menisci may be trapped by the at least one blocking element.

The at least one blocking element may be adapted to narrow the lumen in a proximal direction.

In some cases, the at least one blocking element may be adapted such that it may block a significant amount of the lumen (as seen in a transverse cross section through the catheter (i.e., transverse to the longitudinal/length direction of the catheter) in the proximal direction. In some cases, the at least one blocking element may narrow the lumen by a lesser amount in a distal portion of the catheter as compared to a more proximal portion of the catheter.

In this case, the at least one blocking element may be adapted such that the blocking element may block up 60%, 70%, 80%, 90%, 95% (or any value in between) of the area of the lumen as seen in a cross-sectional view in a proximal portion of the catheter.

Alternatively and analogously, it may also be possible that the at least one blocking element is adapted to only block a fraction of the area of the lumen as seen in a cross-sectional view in a proximal portion of the catheter. In such a case, the at least one blocking element may for example cover 5%, 10%, 20%, 30%, 40%, 50% (or any value in between) of the area of the lumen as seen in a cross-sectional view.

By allowing a rather wide span of the lumen to be blocked by the at least one blocking element, a fine-tuning of the separation of gas and liquid (preferably blood) by means of the at least one blocking element may be enabled.

The at least one blocking element may further be adapted to stop blood from flowing past the at least one blocking element when a relative movement of the inner shaft to the outer shaft occurs.

Since the at least one blocking element may be adapted such that it may trap a blood gas meniscus on the surface of the at least one blocking element, a movement of the blood gas meniscus which may arise from a relative movement of the inner shaft to the outer shaft may be prevented. In a preferred implementation, the blood gas meniscus may only be in contact with either the outer shaft or the inner shaft such that the blood gas meniscus may not be affected by a relative movement of the inner shaft and the outer shaft.

The aforementioned design in particular contributes to an increased safety of the catheter, preventing an accidental movement of the blood gas meniscus into the blood vessel of the patient or to a more proximal portion of the catheter which may thus be clogged.

The at least one blocking element may be adapted in the shape of a wedge, wherein a height of the wedge, measured in a radial direction, increases from a distal to a proximal direction.

By allowing the wedge to increase in height from a distal to a proximal direction, the wedge may narrow a lumen in a distal portion of the wedge by a smaller amount than it narrows the lumen in a more proximal region of the wedge. The amount of narrowing in between a distal most portion of the wedge and a proximal most portion of the wedge may increase linearly, parabolically, etc. with increasing longitudinal distance from a distal-most portion of the wedge.

By adapting the at least one blocking element such that its height increases in a proximal direction of the catheter, it may increase the resistance for a blood gas meniscus for moving in a proximal direction of the catheter the further it moves in a proximal direction. Such an arrangement may additionally contribute to avoiding that a blood gas meniscus may block the lumen in a proximal region of the catheter.

Alternatively, the at least one blocking element may be adapted in the shape of a wedge, wherein a height of the wedge, measured in a radial direction, increases from a proximal to a distal direction.

The at least one blocking element may be adapted to protrude from an inner side of the outer shaft, radially inward towards the inner shaft.

In some cases, the at least one blocking element may be attached to an inner side of the outer shaft. In some cases, the at least one blocking element may be integrally connected to the inner side of the outer shaft.

The shape of the at least one blocking element may not be limited to a wedge like shape. In some cases, the at least one blocking element may be provided in the shape of, e.g., a longitudinally extending rod-like structure. In some cases, the at least one blocking element may protrude in a radial direction (towards the inner shaft) in a straight manner (e.g., without a curvature or a bending of the at least one blocking element). Alternatively, it may also be possible that the at least one blocking element is provided with a curvature (e.g., the at least one blocking element may bend towards a distal or a proximal direction as it protrudes in a radial direction).

By allowing a protrusion of the at least one blocking element from an inner side of the outer shaft, a narrowing of the lumen in a radial direction may be facilitated.

The at least one blocking element may have a circular cross section.

The at least one blocking element may be provided with a circular-shaped cross section as seen from a transverse cut relative to a longitudinal extension of the at least one blocking element. In some cases, the at least one blocking element may relate to a knob-like protrusion. However, if the at least one blocking element protrudes further in a radial direction, the at least one blocking element may be provided as a column-like blocking element. It is noted that the cross-sectional shape of the at least one blocking element is not limited to a circular cross section. In some cases, the column-like blocking element may also be provided with a rectangular cross section.

If at least two blocking elements are implemented, the at least two blocking elements may be of the same type (e.g., wedge-shaped or column-shaped) or may be a combination of one or more possible implementations as described herein.

If the at least one blocking element is provided with a circular cross section, a brush-like feature may be implemented between the inner shaft and the outer shaft which may advantageously and safely retain entrapped air by encumbering the movement of the blood gas meniscus.

The catheter may comprise at least three separate blocking elements.

In some cases, the at least one blocking element may be implemented as at least three separate blocking elements. The at least three blocking elements may be arranged at a same proximal location (e.g., the at least three blocking elements may have the same distance to a distal tip of the catheter) and may be circumferential to the inner shaft while protruding from the inner wall of the outer shaft. In a preferred embodiment, the radial distance between the at least three blocking elements may equal. As an example, if the catheter is provided with three blocking elements, the center of each of the three blocking elements may be angularly separated by 120° from a respective adjacent neighboring blocking element.

By providing the catheter with at least three blocking elements, the overall blocking capability and efficiency in a proximal direction may be increased. Moreover, a symmetrical arrangement of the at least three blocking elements may provide an isotropic blocking against the movement of a blood gas meniscus in a radial plane.

The catheter may comprise at least two blocking elements spaced apart from each other along a longitudinal axis of the catheter.

In some cases, it may be possible that at least two blocking elements are spaced apart from each other such that their separation is, e.g., 1 mm, 5 mm, 1 cm, 2 cm, etc. (or any value in between). In some cases, the at least two blocking elements may be arranged subsequent to each other, i.e., without a relative radial separation to each other. In some cases, the at least two blocking elements may both be spaced apart in the longitudinal direction and in a radial direction (e.g., the more distally located blocking element may be radially separated from the more proximally located blocking element by 120° (or any other angle) or vice versa).

In some cases, it may also be possible that the catheter is provided with a more distally located group of at least two blocking elements (preferably at least three blocking elements) and at least a second more proximally located group of two blocking elements. In such a case, the first group of at least two blocking elements may be spaced apart from the at least second group of at least two blocking elements along a longitudinal axis of the catheter.

By arranging at least two blocking elements along a longitudinal axis of the catheter, the effective filtering capability of the at least two blocking elements for a blood gas meniscus may be improved.

The inner shaft may be adapted to provide a rinsing liquid to a distal end of the inner shaft such that at least a distal portion of the lumen can be rinsed.

The at least distal portion of the lumen may relate to the portion of the lumen which may be adjacent to a distal tip of the inner shaft.

Such an implementation may provide rinsing capabilities of the catheter in a distal-most portion of the catheter.

The at least one blocking element may be adapted to prevent blood from flowing in the proximal direction in a section of the catheter which cannot be rinsed.

Since in some cases, the catheter may not be provided with dedicated means for rinsing in a rather proximal portion of the catheter, such regions may not be rinsed. Therefore, any blood coagulation which may accumulate therein (e.g., due to a blood gas meniscus which may have moved there) may not be rinsed and may avoid a gas transfer in a further proximal direction and may potentially provide a source for a bacterial focus.

By preventing any blood flow in a proximal direction of the catheter, a blocking of a proximal portion of the lumen may be prevented and the establishing of unhygienic circumstances may be suppressed.

Blood flowing in the lumen from a distal end of the catheter may push air, residing in the lumen, in the proximal direction.

The pushing of air in a proximal direction may be based on the inwards flowing blood superseding at least a portion of the air residing in the lumen. Since the only free path for the air is towards the proximal direction, a proximal flow of air may thus be promoted.

By allowing that inflowing blood may supersede air residing in a distal end of the catheter, an escape of air from the catheter into the blood vessel of the patient may be avoided. Any minor amounts of gas (e.g., air), which may still reside in the distal portion, may not be seen as a safety risk for the patient.

Any entrapped air may generally present a minimal risk for escaping into the circulatory system of the patient in which it may create an embolism and, furthermore, the presence of the entrapped air prevents the ingress of blood volumes which, in turn, aids in mitigating the presence of stagnant blood volumes within the catheter (along with their affiliate clotting risks).

The following figures are provided to support the understanding of the present invention:
- Figs. 1A-C: Illustration of an exemplary generic embodiment of a catheter;
- Figs. 2A-C: Illustration of a first exemplary embodiment of a catheter;
- Figs. 3A-B: Illustration of a second exemplary embodiment of a catheter;
- Figs. 4A-C: Illustration of a third exemplary embodiment of a catheter.

The following detailed description outlines possible exemplary embodiments of the invention.

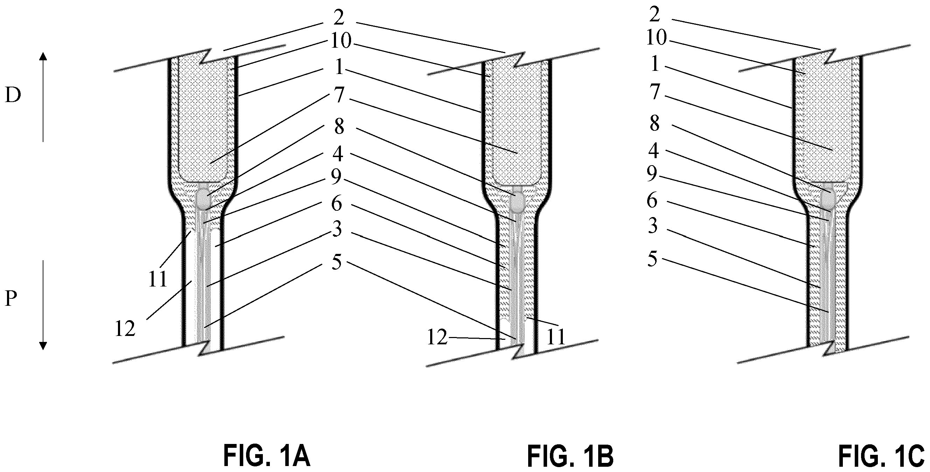

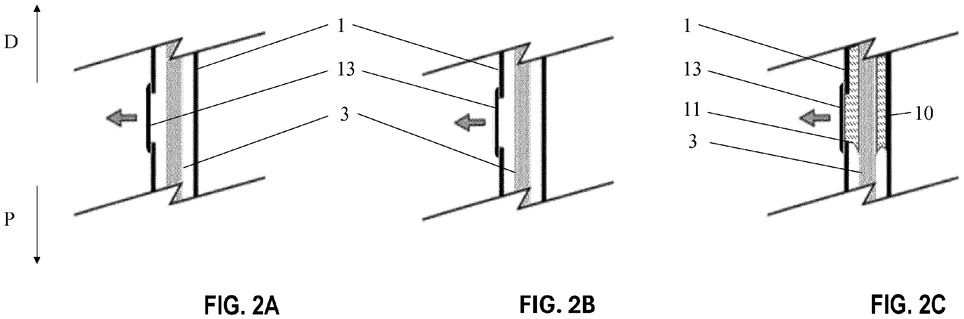

Figs. 1A-C show a generic embodiment of a catheter in a cross-sectional view taken along a longitudinal direction of the catheter. A detailed explanation of further aspects is provided with respect to Figs. 2-4, below.

Fig. 1A shows a catheter extending from a distal direction D to a proximal direction P.

The catheter may comprise an outer shaft 1. The outer shaft 1 may be provided with a diameter which may be larger in a distal portion of the catheter as compared to a proximal portion of the catheter. The outer shaft 1 may be assembled by two tubular-shaped elements with respective diameters. Alternatively, the outer shaft 1 may also be integrally formed with varying diameter (e.g., narrowing towards the proximal direction P). The outer shaft 1 may further comprise an opening 2 on a distal tip of the outer shaft 1. The inner side of the outer shaft 1 may be hydrophilic.

The catheter may further comprise an inner shaft 3 which may be arranged (concentrically) in the outer shaft 1. The inner shaft 3 may predominantly be located in a proximal portion of the outer shaft 1. A distal tip portion of the inner shaft 3 may project into the portion of the outer shaft 1 which is provided with the larger diameter (i.e., the distal portion). Inner shaft 3 may be provided with an opening 4 on a distal end. The interior of the inner shaft 3 may preferably be shallow and may thus have an inner lumen 5. The outer side of the outer shaft 3 may be hydrophilic.

A lumen 6 may be formed in between the outer shaft 1 and the inner shaft 3.

The catheter may further comprise a medical device 7 located or received in a distal portion of the outer shaft 1. The medical device 7 may be provided with attachment means 8.

Attachment means 8 and thus the medical device 7 may be manipulated by manipulator 9. Manipulator 9 may preferable be a wire-like element which extends in the inner lumen 5 of the inner shaft 3 along a proximal direction P of the catheter, preferably to a proximal end of the catheter.

At least a distal portion of the outer shaft 1 may be partially filled with a liquid 10 (e.g., blood which may have entered the outer shaft 1 by means of the opening 2 and/or a rinsing fluid which may have been provided to the distal portion of the outer shaft 1 by means of the inner lumen 5 of the inner shaft 3).

Due to the hydrophilic properties of the inner side of the outer shaft 1 and the outer side of the inner shaft 3, the liquid 10 may be pulled in a proximal direction P of the catheter and may form a meniscus 11 in a proximal portion of the lumen (in between the inner side of the outer shaft 1 and the outer side of the inner shaft 3).

At least a proximal portion of the catheter, which is not yet filled with the liquid 10, accommodate a gas 12, preferably air.

Fig. 1B depicts the catheter of Fig. 1A wherein a larger volume of liquid 10 has been pulled into the lumen 6 and further along a proximal direction P of the catheter.

Said pulling of the liquid 10 may at least in part be based on forces (e.g., capillary forces) acting in the narrow lumen between the outer shaft 1 and the inner shaft 3.

As a result of the larger volume of liquid 10, which has been pulled into the lumen 6, the volume of the gas 12 may be pushed into the proximal direction P of the catheter. In some cases, the volume of the gas 12 may have been decreased (as an effect of a nominal compression if the gas 12 cannot exit the catheter) based at least in part on the additional liquid 10 which has been flown into the lumen 6.

Fig. 1C depicts the catheter of Figs. 1A and 1B wherein an even larger volume of liquid 10 has been pulled into the lumen 6 and further along a proximal direction P of the catheter.

Fig. 1C shows a situation in which the entire lumen 6 is filled with the liquid 10 up to a proximal portion of the catheter.

In this scenario, no gas 12 may be left in the proximal portion of the catheter.

Figs. 2A-C show a detailed view of a proximal portion of the catheter proximal to the distal portion of the catheter as depicted in and described with reference to Figs. 1A-C in a cross-sectional view (taken along a longitudinal axis of the catheter). Figs. 2A-C are to be understood as any proximal portion of the catheter proximal to the distal portion of the catheter as depicted in Figs. 1A-C, respectively. The depicted portion in Figs. 2A-C may be for example the proximal-most portion of the catheter, which is for example functionally connected to a handle of the catheter It is emphasized that aspect described with reference to Figs. 2A-C may be combined with aspects described with reference to Figs. 1A-C.

The proximal portion of the outer shaft 1 of the catheter may comprise a proximal opening 13, which may preferably be impermeable for the liquid 10 but permeable for the gas 12.

More specifically, Fig. 2A shows a situation which corresponds to the situation shown in Fig. 1A, in which only a minor volume of the liquid 10 has been flown into the catheter. Therefore, the depicted proximal portion is free of the liquid 10.

Fig. 2B shows a situation which corresponds to the situation shown in Fig. 1B in which a larger volume of the liquid 10 has been pulled into the lumen 6. However, the liquid 10 has not been pulled in the depicted proximal portion of the catheter. Therefore, Fig. 2B does not depict the liquid 10.

Fig. 2C shows a situation which corresponds to the situation shown in Fig. 1C in which an even larger volume of the liquid 10 has been pulled into the lumen 6. In this case, the liquid 6 has even been pulled in the depicted proximal portion of the catheter.

When being pulled in the depicted proximal portion of the catheter, the liquid 10 may push the gas 12 into the proximal portion of the catheter.

The proximal opening 13 may be adapted to allow the gas 12 to exit the catheter through the outer shaft 1 while preventing the liquid 10 from exiting the outer shaft 1 through the proximal opening 13.

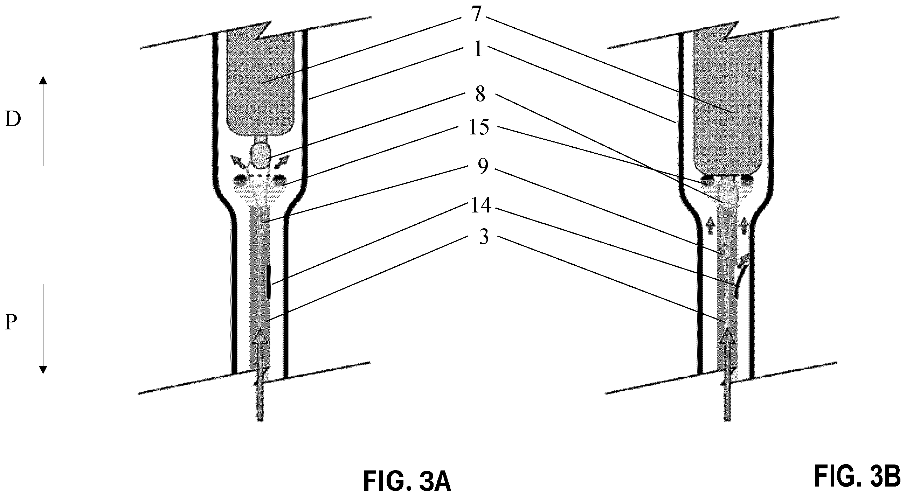

Figs. 3A-B show another embodiment of a catheter in a cross-sectional view (taken along a longitudinal axis of the catheter) in which the catheter may be provided with two predetermined configurations. The catheter described with reference to this embodiment is shown in Figs. 3A-B and may correspond to the catheter depicted in Figs. 1A-C and Figs. 2A-C discussed above. It is emphasized that aspects described with reference to Figs. 3A-B may be combined with aspects described with reference to the aforementioned figures.

Fig. 3A shows the catheter in a second predetermined configuration.

As described above, the catheter may comprise the outer shaft 1 which accommodates the inner shaft 3. In the outer shaft 1, the medical device 7 may be located, particularly in distal tip or most distal portion of the outer shaft 1.

According to an aspect, the inner shaft 3 may be provided with a pressure sensitive valve 14, which may allow a fluid communication between the inner lumen 5 of the inner shaft 3 and an inner lumen of the outer shaft 1.

Medical device 7 may be provided with an attachment means 8 which may be in contact with manipulator 9.

Manipulator 9 may be located in the inner shaft 3 and may extend to the proximal direction P of the catheter.

At a distal end of the inner shaft 3, a funnel-like element 15 may be located. The funnel-like element 15 may be adapted to be (at least liquid-tightly) engageable with the medical device 7 and may be in fluid communication with a distal end of the inner lumen 5 of the inner shaft 3.

However, in the depicted exemplary scenario, the funnel-like element 15 does not engage with the medical device 7. Therefore, any liquid filled into the inner lumen 5 of the inner shaft 3 may exit the inner shaft 3 through the funnel-like element 15 and may further flow towards the distal direction D. Thereby, the distal portion of the catheter may be rinsed.

Fig. 3B corresponds to the catheter shown in Fig. 3A, however, the funnel-like element 15 is depicted as (liquid-tightly) engaged with the medical device 7 thus putting the catheter into the first predetermined configuration.

Therefore, any liquid filled into the inner lumen 5 of the inner shaft 3 may thus arrive at a blocked distal tip of the inner shaft 3. If further liquid is filled into the inner lumen 5 of the inner shaft 3, an increase of pressure in the inner lumen 5 of the inner shaft 3 may be the initiated. Said increase in pressure may result in an opening of the pressure sensitive valve 14 located on the inner shaft 3 if the pressure inside the outer shaft 3 exceeds a predetermined value.

In ideal operation, a physician may flush a distal portion of the lumen 6 by means of the inner shaft 3 and the catheter being set to each of the first predetermined configuration and the second predetermined configuration. Such a procedure may remove entrapped air from the accessible portions of the lumen 6. Following the insertion of such a catheter in the patient's vasculature, a similar administration of the first predetermined configuration and the non-predetermined configuration may then enable removal of stagnant blood thus avoiding unwanted risks of thrombi.

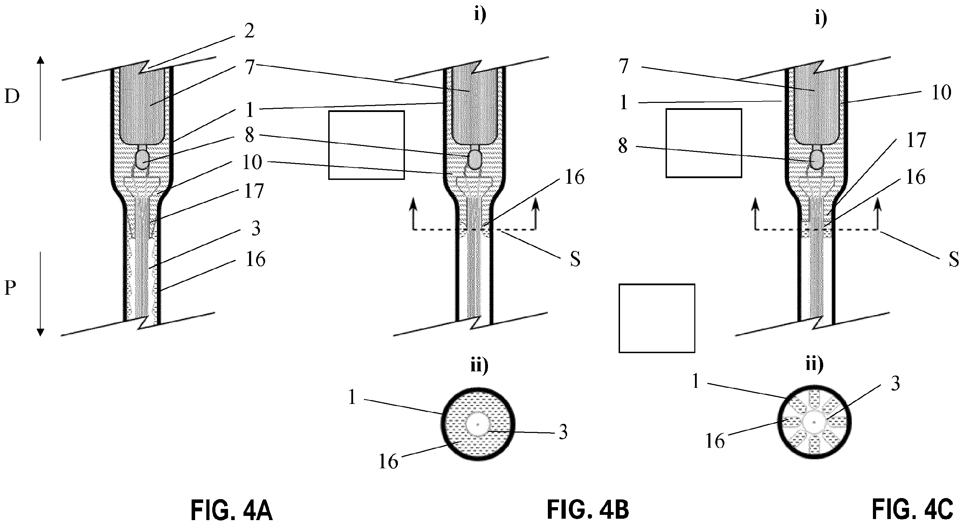

Figs. 4A-C relate to a further embodiment of the catheter, wherein the catheter is shown in a longitudinal cross-sectional view. The catheters depicted in Figs. 4A-C correspond to the catheters as described above with reference to Figs. 1A-C, Figs. 2A-C and Figs. 3A-B. It is emphasized that aspects described with reference to Figs. 4A-B may be combined with aspects described with reference to the aforementioned figures. Special features of the catheters depicted in Figs. 4A-C will be described below in further detail.

Figs. 4A-C depict various exemplary mechanisms how to prevent blood, which may enter the catheter (and more specifically the outer shaft 1) through the distal opening 2.

The catheter may be provided with at least one blocking element 16 which is preferably located in a proximal portion of the catheter (the outer shaft 1, respectively). The at least one blocking element may be adapted to narrow the lumen 6, defined in between the outer shaft 1 and the inner shaft 3.

The at least one blocking element 16 may be provided in one or more out of several possible shapes (as seen in a longitudinal cross-sectional view). For instance, the at least one blocking element may be provided in the shape of a wedge (Fig. 4A) or at least wedge-like.

The at least one blocking element 16 may be adapted such that it may stop a blood gas meniscus 17 from moving/migrating in the proximal direction P. Therefore, the at least one blocking element 16 may act as a filter which may prevent an undesired movement of the blood gas meniscus 17 in the proximal direction P or the distal direction D while, preferably, still allowing the gas 12 to pass the at least one blocking element 16 in the proximal direction P, wherein the gas 12 is preferably drawn from the outer shaft 1 by a proximal opening 13 (not shown in Figs. 4A-C), wherein the proximal opening 13 is preferably located at a proximal portion being outside of the patient, e.g. the most proximal portion of the outer shaft 1, e.g. being functionally connected to a handle of the catheter .

As further depicted in Fig. 4A, there may be multiple blocking elements 16, spaced apart from each other in the further proximal direction P.

Fig. 4B i) depicts a different embodiment of the at least one blocking element 16, which however, may be combined with the potential embodiment of the at least one blocking element 16 as described with reference to Fig. 4A, above.

The at least one blocking element 16 may also be implemented as an O-ring-like element. The O-ring-like element may be adapted to bridge between the inner side of the outer shaft 1 and the outer side of the inner shaft 3. The O-ring-like element may preferably concentrically accommodate the inner shaft 3.

The O-ring-like element may thus allow the catheter to maintain a separation of the gas 12 (e.g., air) and the liquid 10 (e.g., blood) in targeted volumes (e.g., it may be preferable to retain gas in a proximal portion of the catheter whereas the liquid may be retained in the distal portion of the catheter). In other words, the O-ring-like element may act as a seal in between the distal portion of the catheter (which may, e.g., be filled with blood) and the proximal portion of the catheter (which may, e.g., be filled or at least in contact with the gas 12 (e.g., air)). The sealing properties of the O-like-element may even be maintained under a relative (longitudinal) motion of the outer shaft 1 and the inner shaft 3. Therefore, a physical segmentation of the catheter into distinct gas- and blood-only regimes may be achieved.

Fig. 4B ii) depicts a cross-sectional view of a proximal portion of the catheter, taken transverse to the longitudinal extension of the catheter and along line S.

Fig. 4B ii) shows how the at least one blocking element 16, when arranged in an O-ring-like manner may protrude, e.g., from an inner side of the outer shaft 3 in a radial direction towards the outer side of the inner shaft 3.

The at least one blocking element 16 may be adapted such that it may fill the entire lumen 6, defined in between the outer shaft 1 and the inner shaft 3. Alternatively, the at least one O-ring-like blocking element 16 may also be adapted such that it only partially fills the lumen 6.

Fig. 4C i) shows another embodiment of the at least one blocking element 16 which may also be combinable with the embodiments of the at least one blocking element 16 as described with reference to Figs. 4A and 4B, above.

Fig. 4C i) shows an embodiment in which the at least one blocking element 16 may be implemented as an element protruding from, e.g., an inner side of the outer shaft 1, which may narrow the proximal portion of the lumen 6. If more than one blocking element 16 protrudes from the inner side of the outer shaft 1, a tendril/finger-like or brush-like structure may be formed, respectively. The achievable narrowing of the lumen 6 may serve to amplify the effects associated with the surface tension at the blood/gas boundary such that predominantly capillary effects create forces which may be inclined to push the air/gas boundary into an air-entrapped portion of the catheter. However, said capillary effects may, in some cases, pull the blood gas meniscus 17 further towards the proximal direction P into a region of the catheter which should not be entered by the blood gas meniscus 17. Therefore, the at least one blocking element 16, when implemented according to this embodiment, the forcing of the blood gas meniscus into a further proximal portion of the lumen 6 may be balanced by a counteraction of adhesion/friction effects supplied by the at least one blocking element 16 such that a further movement of the blood gas meniscus 17 may be impeded. Said effect may additionally be enhanced if the at least one blocking element 16 is provided with a textured surface.

To understand this surface effect that "grabs" the blood gas meniscus 17 to challenge its migration, one can dip a coarse-grit piece of sandpaper into a glass of water to see bubbles on the surface of the grit that tend to hold in place but track the movement of the paper.

As described beforehand, an essentially physical segmentation of the catheter into gas- and blood-only regimes may be facilitated by means of using restrictive geometries (in analogy to the restriction described with reference to Fig. 4A, above).

Fig. 4C ii) shows a cross-sectional view of a proximal portion of the catheter, taken transverse to the longitudinal extension of the catheter and along line S.

The at least one blocking element 16 may be adapted as respective one or more protrusions, protruding in a radial direction towards the outer side of the inner shaft 3 and preferably originating from the inner side of the outer shaft 1.

As outlined beforehand, the potential blood gas meniscus 17, moving in the proximal direction P may arrive at the brush like structure, which may be formed by the at least one blocking element 17 according to this embodiment, and may thus be impeded from a further movement in the proximal direction P.

To summarize, the at least one blocking element 16, in each of the aforementioned embodiments, may be adapted to hold the blood gas meniscus in a stable location and prevent a movement in a proximal direction P. It should be noted that lacking a means to instate a pressure gradient across the entrapped air, its expulsion has no driving force. As such, when paired with the incorporated physical interference features noted above, air escape and blood ingress are unlikely.

## Claims

1. A catheter, comprising:
an inner shaft (3) and an outer shaft (1) such that a lumen (6) is defined therebetween;
wherein the lumen (6) comprises a distal opening (2) and a proximal opening (13; 14);
wherein the proximal opening (13; 14) is configured such that a gas (12) in the lumen (6) can leave the outer shaft (1) via the proximal opening (13; 14); and
wherein the proximal opening (13; 14) is configured to be impermeable for a liquid (10).

2. The catheter of claim 1, wherein the distal opening (2) is configured such that a fluid can enter the lumen (6) via the distal opening (2).

3. The catheter according to claim 1 or 2, wherein a distance between the inner shaft (3) and the outer shaft (1) is sufficiently small such that a liquid (10) at the distal opening (2) is pulled into the lumen in a proximal direction (P).

4. The catheter according to claim 3, wherein the liquid (10) is a rinsing liquid.

5. The catheter according to claim 4, wherein the catheter is adapted such that the rinsing liquid can be delivered into the lumen (6) by insertion into an inner lumen (5) of the inner shaft (3).

6. The catheter according to one of the claims 6 or 7, wherein the catheter is adapted such that the rinsing liquid at least partially reduces the inflow of blood into the lumen (6).

7. The catheter according to any of the previous claims, wherein an outer side of the inner shaft (3) and/or an inner side of the outer shaft (1) is hydrophilic.

8. The catheter according to any of the previous claims, wherein the inner shaft (3) and the outer shaft (1) are movable/slidable relative to each other at least along a longitudinal extension of the inner shaft (3) and the outer shaft (1).

9. The catheter according to any of the previous claims, wherein the catheter is configured such that a proximal flow of liquid (10) within the lumen (6) causes gas in the lumen (6) to be pushed out of the lumen (6) via the proximal opening (13; 14).

10. The catheter according to claim 9, wherein the proximal opening (13; 14) comprises a venting structure, preferably a one-way venting structure.

11. The catheter according to any of the previous claims, wherein a distance between the inner shaft (3) and the outer shaft (1) is adapted such that a blocking of the lumen by stagnant blood is avoided.

12. The catheter according to any of the previous claims, wherein the proximal opening (13; 14) resides in a handle of the catheter.

13. The catheter according to any of the previous claims, wherein the proximal opening (13; 14) comprises a polymer.

14. The catheter according to any of the previous claims, wherein the outer shaft (1) is configured as an outer surface of the catheter and/or the lumen (6) is adapted to be directly adjacent to an inner lumen (5) of the inner shaft (3).

15. The catheter according to any of the previous claims, wherein the catheter is further adapted to accommodate a medical device (7) distally to the lumen.
